# EUROPÄISCHE PATENTSCHRIFT

(11) **EP 0 652 750 B1**
(45) Veröffentlichungstag und Bekanntmachung des Hinweises auf die Patenterteilung: **17.12.1997**
(21) Anmeldenummer: 93915948.9
(22) Anmeldetag: 17.07.1993
(51) Int. Cl.: A61K 31/20

(54) **VERWENDUNG VON ALPHA,OMEGA-DICARBONSÄUREN ALS FRIBINOGENSENKER**
USE OF ALPHA,OMEGA-DICARBOXYLIC ACIDS AS FIBRINOGEN REDUCERS
UTILISATION D'ACIDES ALPHA,OMEGA-DICARBOXYLIQUES COMME FIBRINOGENOREDUCTEURS

(30) Priorität: 25.07.1992 DE 4224670
(43) Veröffentlichungstag der Anmeldung: 17.05.1995
(73) Patentinhaber: YISSUM RESEARCH DEVELOPMENT COMPANY OF THE HEBREW UNIVERSITY OF JERUSALEM, Jerusalem 91042 (IL)
(72) Erfinder: PILL, Johannes, D-69181 Leimen (DE); DOERGE, Liesel, D-68259 Mannheim (DE); STEGMEIER, Karlheinz, D-64646 Heppenheim (DE)
(74) Vertreter: Weisert, Annekäte, Dipl.-Ing. Dr.-Ing.
(86) Internationale Anmeldenummer: EP9301894
(87) Internationale Veröffentlichungsnummer: WO9402128

(56) Entgegenhaltungen:
- EP-A- 0 081 930
- WO-A-88/02746
- TRENDS IN PHARMACOLOGICAL SCIENCE Bd. 11, Nr. 11 , November 1990 Seiten 444 - 451 COOK, N.S. ET AL 'FIBRINOGEN AS A MAJOR RISK FACTOR IN CARDIOVASCULAR DISEASE' in der Anmeldung erwähnt
- PHARMACOLOGY Bd. 23, Nr. 5 , 1981 Seiten 271 - 280 PICKART, L. 'FAT METABOLISM, THE FIBRINOGEN/FIBRINOLYTIC SYSTEM AND BLOOD FLOW: NEW POTENTIALS FOR THE PHARMACOLOGICAL TREATMENT OF CORONARY HEART DISEASE'
- JOURNAL OF MEDICINAL CHEMISTRY Bd. 32, Nr. 9 , September 1989 Seiten 2072 - 2084 'SYNTHESIS AND HYPOLIPIDAEMIC AND ANTIDIABETOGENIC ACTIVITIES OF BBBB-TETRASUBSTITUTED, LONG-CHAIN DIOIC ACIDS'

## Beschreibung

Die vorliegende Erfindung betrifft die Verwendung von α,w-Dicarbonsäuren zur Herstellung von Arzneimitteln zur Verhinderung und Behandlung von Fibrinogen-mediierten obstruktiven Vorgängen in Gefäßen.

Fibrinogen ist ein Glycoprotein im Blut, das eine essentielle Rolle in der Hämostase und Aufrechterhaltung der Blutviskosität spielt. Es ist an der Bildung von Plättchenaggregaten und in der Gerinnungskaskade beteiligt. Durch Thrombin wird Fibrinogen in Fibrinmonomere überführt, die nach Polymerisation die Basis für hämostatische Verschlüsse bilden.

Fibrinogen ist neben anderen ein unabhängiger Risikofaktor (J. Intern. Med. 227, 365-372, 1990) für cardiovasculäre Erkrankungen, wie in verschiedenen großen prospektiven klinischen Studien gezeigt werden konnte (zusammengefaßt in TiPS 11, 444-451, 1990). Erhöhte Plasmafibrinogenspiegel sind häufig mit einer Dyslipidämie assoziiert, aber auch mit peripherer vaskulärer Verschlußkrankheit, Hypertension oder Diabetes (J. Am. Med. Assoc. 258, 1183 - 1186, 1987; J. Am. Med. 85, 584-585, 1988). Die Verschlußrate nach Bypassoperation ist ebenfalls mit der Höhe des Plasmafibrinogens korreliert (Brit. Med. J. 299, 643-646, 1989). Es ist allgemein akzeptiert, daß erhöhte Fibrinogenspiegel zu morphologischen Veränderungen in den Gefäßen führen und nicht eine Folge dieser sind (J. Intern. Med. 227, 365-372, 1990).

Wirkstoffe, die zu einer Erniedrigung des Plasmafibrinogens führen, sind bei einer Reihe von pathologischen Zuständen daher von therapeutischem Wert.

Der vorliegenden Erfindung liegt die Aufgabe zugrunde, geeignete Arzneimittel bereitzustellen, die zur Behandlung der Hyperfibrinogenämie verwendet werden können.

Im Rahmen der vorliegenden Erfindung wurde nun überraschenderweise gefunden, daß α,w-Dicarbonsäuren der nachstehenden Formel I zu einer Erniedrigung des Fibrinogens im Plasma bei verschiedenen Tierspezies führen bzw. den Anstieg des Fibrinogens im Blutplasma nach Stimulation abschwächen. Die Substanzen eignen sich daher zur Behandlung bzw. Prävention von Hyperfibrinogenämien, in deren Folge obstruktive Gefäßerkrankungen auftreten wie z.B. bei arteriosklerotischen Veränderungen, Erkrankungen des venösen Systems mit der Folge thrombotischer und embolischer Komplikationen oder Hyperfibrinogenämien nach angioplastischen Maßnahmen und entzündlichen oder neoplastischen Prozessen.

Gegenstand der vorliegenden Erfindung sind α,w-Dicarbonsäuren der allgemeinen Formel I 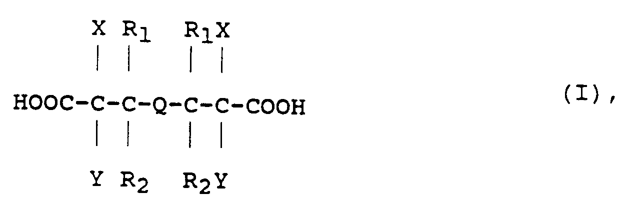 in der
- X und Y,: die gleich oder verschieden sein können, Wasserstoff, Halogen, C₁-C₆-Alkyl, C₁-C₆-Alkoxy, Hydroxy, Cyano, Carboxyl, C₁-C₆-Alkoxycarbonyl oder Carbamoyl,
- R₁ und R₂,: die gleich oder verschieden sein können, Wasserstoff oder C₁-C₆-Alkyl, und
- Q: eine lineare gesättigte oder ungesättigte Alkylenkette mit 2-14 C-Atomen, in der ein oder mehrere C-Atome durch Cycloalkylringe, Phenyl oder Heterocyclen ersetzt sein können,
bedeuten,
sowie deren in-vivo hydrolisierbare Carbonsäure-Derivate.

Bevorzugt sind Verbindungen der Formel I, in der
- X und Y,: die gleich oder verschieden sein können, Wasserstoff oder Halogen,
- R₁ und R₂,: die gleich oder verschieden sein können, Wasserstoff oder Methyl, und
- Q: eine lineare Kette mit 2-14 C-Atomen, eine (CH₂)ₙ Cyclohexyliden-(CH₂)ₙ-, eine (CH₂)ₘ-Phenylen-(CH₂)ₘ-, eine CH₂-CH=CH-Phenylen-CH=CH-CH₂- oder eine -CH₂-CH=CH-CH₂-Phenylen-CH₂-CH=CH-CH₂-Gruppe, wobei n die Zahlen 2, 3 oder 4 und m die Zahlen 3 oder 4 darstellen, bedeutet, sowie deren in-vivo hydrolisierbare Carbonsäure-Derivate.

Insbesondere bevorzugt sind die beiden Verbindungen der Formel I, in denen X und Y jeweils Wasserstoff, R₁ und R₂ jeweils eine Methylgruppe und Q eine (CH₂)₁₀-Gruppe (Verbindung A) und X und Y jeweils Chlor, R₁ und R₂ jeweils Wasserstoff und Q eine (CH₂)₈-Gruppe (Verbindung B) bedeuten.

Unter Halogen bei der Bedeutung von X bzw. Y ist Fluor, Chlor oder Brom zu verstehen, insbesondere Chlor und Brom.

Cycloalkylringe sind carbocyclische Ringe mit 3-7 C-Atomen, bevorzugt ist der Cyclohexyl-Ring.

Hetereocyclen sind vorzugsweise Piperidin-, Piperazin- und Pyrrolidin-Ringe.

Das Phenyl-, Cyclohexyl-, Piperidin- sowie das Piperazin-Ringsystem der Kette Q kann über die 1,2-, 1,3- oder 1,4-Stellung in die Kette eingebaut sein.

α,w-Dicarbonsäuren der Formel I sind Gegenstand der Patentanmeldungen EP-A-81 930, EP-A-185 080 und EP-A-279 978,

in denen die Verbindungen als Arzneimittel zur Behandlung von Fettleibigkeit, Hyperlipidämie oder Diabetes beschrieben werden.

Die Verbindungen der Formel I werden nach den in den zitierten Patentanmeldungen aufgeführten Verfahren hergestellt.

Die pharmakologischen Untersuchungen hinsichtlich einer fibrinsenkenden Wirkung werden beispielhaft für die beanspruchten α,w-Dicarbonsäuren mit der oben angeführten Verbindungen A und B durchgeführt.

Zur Behandlung und Prävention einer Hyperfibrinogenämie zur Verhinderung obstruktiver Gefäßerkrankungen werden α,w-Dicarbon-säuren systemisch, vorzugsweise enteral, besonders bevorzugt oral, aber auch parenteral (i.v.) verabreicht. Die Dosis variiert gemäß den Bedürfnissen des einzelnen Patienten, wie sie vom behandelnden Arzt bestimmt worden ist. Für die Verbindung B dürfte im allgemeinen eine tägliche Dosis von etwa 100 - 1000 mg verwendet werden. Dieser Dosisbereich kann aufgrund der tierexperimentellen Daten als ausreichend angesehen werden. Zur Verhinderung einer Restenose nach Angioplastie sollte die α,w-Dicarbonsäure vorzugsweise vor der Intervention gegeben werden und danach solange, bis das Risiko einer Restenosierung vernachlässigbar geworden ist. In diesem Zusammenhang soll auch noch darauf hingewiesen werden, daß wegen der Möglichkeit der oralen Verabreichung der α,w-Dicarbonsäuren eine Behandlungsdauer zeitlich nicht begrenzt ist, was für die erfindungsgemäße Verwendung wesentlich ist.

Als Verabreichungsform kommen die für die systemische Verabreichung üblichen festen oder flüssigen Darreichungsformen in betracht, z.B. Suppositorien oder als feste orale Darreichungsformen, Kapseln, Tabletten, Lacktabletten, Dragees, Pillen, Puder, Granulate und dergl., als flüssige orale Darreichungsformen Lösungen, Sirupe, Suspensionen, Elexiere und dergl. und als parenterale Darreichungsformen Infusions- oder Injektionslösungen, die i.v. oder i.m. injiziert werden können.

Es liegt im Bereich der vorliegenden Erfindung, die α,w-Dicarbonsäuren in jeder für die Verabreichung geeigneten Menge in die enterale oder orale Darreichungsform einzuarbeiten. Es ist jedoch bevorzugt Präparate herzustellen, die pro Dosierungseinheit den Wirkstoff in einer Menge bis zu 1000 mg vorzugsweise von etwa 100 - 400 mg enthalten. Besonders bevorzugt ist die Herstellung von Kapseln, Tabletten und Lacktabletten als Dosierungseinheit. Diese können nach den jeweiligen vom Arzt festzulegenden Erfordernissen ein- oder mehrmals täglich appliziert werden.

Die Herstellung der oben genannten Gebrauchsformen kann z.B. anhand des nachstehenden Beispiels für Verbindung B erfolgen.

### Beispiel 1: Herstellung einer Tablettier- oder Kapselfüllmasse

| Tabletten mit 200 mg Verbindung B | |
|---|---|
| Zusammensetzung | mg/Kapsel |
| 1. Verbindung B | 200.0 |
| 2. Lactose x 1H₂O | 50.0 |
| 3. Crospovidon | 10.0 |
| 4. Povidon 25.000 | 10.5 |
| 5. Crospovidon | 5.0 |
| 6. Siliziumdioxyd kolloidal | 2.5 |
| 7. Zellulose mikrokristallin | 20.0 |
| 8. Magnesiumstearat | 5.0 |
| Füllgewicht | 303.0 |
| 9. Gelatinekapseln | Größe 0 |

Die Bestandteile werden miteinander nach herkömmlichen Verfahren gemischt und feucht oder trocken granuliert. Die fertige Masse kann zu Kernen verpreßt und direkt als Tabletten oder mit einem Film überzogen als Filmtabletten verwendet werden. Die Masse kann auch direkt in Kapseln, wie z.B. Gelatinekapseln, abgefüllt werden.

### Beispiel 2: Therapeutische Wirkung

Die therapeutische Wirkung von Verbindung A und Verbindung B zur Senkung von Fibrinogen im Blutplasma kann den nachfolgenden Versuchen entnommen werden. Die Versuche sind so beschrieben, daß jedermann, der die nötigen Kenntnisse und Einrichtungen besitzt, sie nachvollziehen kann. Es ist nicht beabsichtigt, durch die Auswahl der durchgeführten Experimente in irgendwelcherweise den Bereich der Anwendung der Erfindung zu beschränken.

### 1a) Allgemeines Prinzip

Die Verabreichung von Terpentin i.m. führt bei der Ratte zu einer drastischen Erhöhung des Fibrinogens im Plasma nach 24 Stunden (168 auf 490 mg/100 ml). Dieser Anstieg ist im wesentlichen durch eine Steigerung der endogenen Synthese bedingt (Clin. Hemorheol 11, 465-477, 1991). Eine Substanzwirkung läßt sich gut an einer Abschwächung der Terpentin-bedingten Hyperfibrinogenämie nachweisen.

### 1b) Versuchsbeschreibung

Männliche Sprague-Dawley-Ratten erhielten über 4 Tage Verbindung A oder B in einer Dosierung von 25 mg/kg/d in 1%iger Tylose-Suspension. Die Kontrollen erhielten die gleiche Menge des Vehikels. Am 4. Tag wurde unmittelbar vor der Substanzapplikation Blut zur Bestimmung des Fibrinogens aus der Schwanzvene entnommen. Zusätzlich wurde 1 ml Terpentin/kg i.m. verabreicht. 24 Stunden nach Applikation von Terpentin wurde erneut Blut aus der Schwanzvene zur Bestimmung von Fibrinogen entnommen.

### 1c) Ergebnisse

Während bei den Lösungsmittel-behandelten Kontrollen das Fibrinogen im Blutplasma im Mittel um 322 mg/100 ml (n = 6) ansteigt, wurde bei den mit Verbindung B- oder Verbindung A-behandelten Tieren eine Erhöhung um 222 bzw. 225 mg/100 ml (n = 12 bzw. 6) gefunden.

### 2a) Allgemeines Prinzip

Genetisch hypercholesterinämische Kaninchen, die spontan eine Atherosklerose entwickeln, haben erhöhte Plasmafibrinogenspiegel (Thromb. Haemost. 61, 140-143, 1989). Wir untersuchten, ob Verbindung B in diesen Tieren im Vergleich zu anderen lipidsenkenden Substanzen wie Probucol und Mevinolin zu einer Senkung des Fibrinogens im Plasma führt.

### 2b) Versuchsbeschreibung

Männliche genetisch hypercholesterinämische (WHHL) Kaninchen erhielten über einen Zeitraum von 8 Wochen Futter, dem 0.33 % Verbindung B, 1 % Probucol oder 0.033 % Mevinolin zugemischt war. Die Kontrolltiere erhielten substanzfreies Futter. Unmittelbar vor sowie nach 8wöchiger Behandlung wurde den Tieren aus der zentralen Ohrarterie Blut zur Fibrinogen-Bestimmung entnommen.

### 2c) Ergebnisse

In der folgenden Tabelle sind die Fibrinogen-Konzentrationen im Blutplasma vor Behandlungsbeginn und nach 8wöchiger Behandlung wiedergegeben. Die mit Verbindung B behandelten Tiere

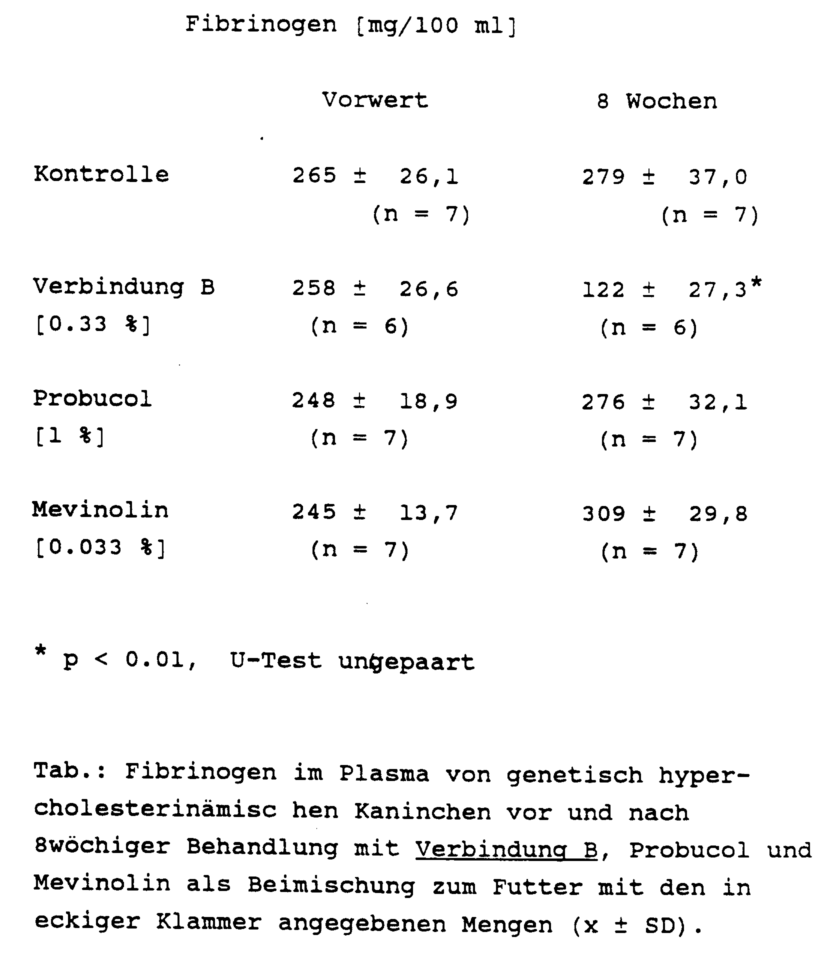

zeigen einen statistisch signifikant niedrigeren Fibrinogenspiegel im Plasma, während die Kontrolltiere sowie die mit Probucol und Mevinolin behandelten im Mittel leicht höhere Werte aufweisen.

### 3a) Allgemeines Prinzip

Zur Evaluierung der Übertragbarkeit der bei Ratte una Kaninchen beobachteten Fibrinogensenkung von Verbindung B auf andere Spezies wurde Fibrinogen bei Hunden nach 3monatiger Substanz-Behandlung bestimmt.

### 3b) Versuchsbeschreibung

Männliche Beagle-Hunde erhielten über einen Zeitraum von 3 Monaten Verbindung B in Dosen von 7, 15 und 35 mg/kg/d in Form von Ovula. Am Ende der Behandlung wurde Blut aus der Vorderbeinvene zur Bestimmung von Fibrinogen entnommen.

### 3c) Ergebnisse

Aus der folgenden Tabelle ist zu entnehmen, daß Verbindung B bereits in einer Dosis von 7 mg/kg/d beim Hund zu einer statistisch signifikanten Erniedrigung des Fibrinogens im Blutplasma führt. Die weitere Erhöhung der Dosis von Verbindung B auf 15 oder 35 mg/kg/d führt zu keiner Verstärkung der Fibrinogensenkung.

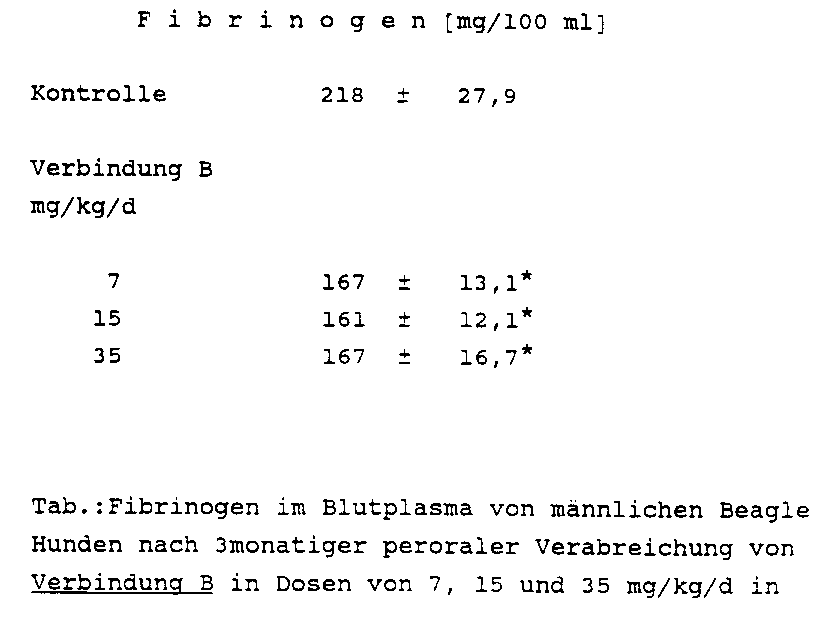

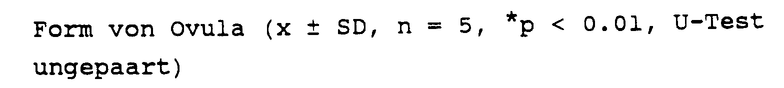

Die hier aufgeführten Ergebnisse belegen eindeutig, daß α,w-Dicarbonsäuren eine Fibrinogen-senkende Wirkung besitzen. Dieser Effekt ist - wie vergleichende Untersuchungen mit den Lipidsenkern Probucol und Mevinolin zeigen - nicht der bereits beschriebenen hypolipidämischen Wirkung von α,w-Dicarbonsäuren zuzuschreiben, sondern stellt eine neue Wirkqualität dieser Substanzklasse dar.

## Patentansprüche

1. Verwendung von α,w-Dicarbonsäuren der Formel I 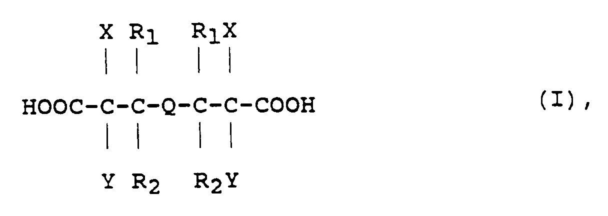 in der
X und Y, die gleich oder verschieden sein können, Wasserstoff, Halogen, C₁-C₆-Alkyl, C₁-C₆-Alkoxy, Hydroxy, Cyano, Carboxyl, C₁-C₆-Alkoxycarbonyl oder Carbamoyl,
R₁ und R₂, die gleich oder verschieden sein können, Wasserstoff oder C₁-C₆-Alkyl, und
Q eine lineare gesättigte oder ungesättigte Alkylenkette mit 2-14 C-Atomen, in der ein oder mehrere C-Atome durch Cyclohexylringe, Phenyl oder Heterocyclen ersetzt sein können,
bedeuten,
sowie deren in-vivo hydrolisierbare Carbonsäure-Derivate zur Herstellung von Arzneimitteln mit Fibrinogensenkender Wirkung.

2. Verwendung gemäß Anspruch 1, dadurch gekennzeichnet, daß man eine Verbindung der Formel I einsetzt, in der
X und Y, die gleich oder verschieden sein können, Wasserstoff oder Halogen,
R₁ und R₂, die gleich oder verschieden sein können, Wasserstoff oder Methyl, und
Q eine lineare Kette mit 2-14 C-Atomen, eine (CH₂)-ₙ-Cyclohexyliden-(CH₂)ₙ-, eine (CH₂)ₘ-Phenylen-(CH₂)ₘ-, eine CH₂-CH=CH-Phenylen-CH=CH-CH₂- oder eine -CH₂-CH=CH- CH₂-Phenylen-CH₂-CH=CH-CH₂-Gruppe, wobei n die Zahlen 2, 3 oder 4 und m die Zahlen 3 oder 4 darstellen bedeutet, sowie deren in-vivo hydrolisierbare Carbonsäure-Derivate.

3. Verwendung gemäß Anspruch 1 oder 2, dadurch gekennzeichnet, daß man eine Verbindung der Formel I einsetzt, in der X und Y jeweils Wasserstoff, R₁ und R₂ jeweils eine Methylgruppe und Q eine (CH₂)₁₀-Gruppe (Verbindung A) und X und Y jeweils Chlor, R₁ und R₂ jeweils Wasserstoff und Q eine (CH₂)₈ -Gruppe (Verbindung B) bedeuten.

4. Verwendung von Verbindungen der Formel I gemäß Anspruch 1, 2 oder 3 zur Herstellung von Arzneimitteln zur Reduktion von Fibrinogen im Blut sowie zur Behandlung und Prävention von Hyperfibrinogenämien.

5. Verwendung von Verbindungen der Formel I gemäß Anspruch 1, 2 oder 3 zur Herstellung von Arzneimitteln zur Verhinderung Fibrinogen-mediierter Gefäßveränderungen.

6. Verwendung von Verbindungen der Formel I gemäß Anspruch 1, 2 oder 3 zur Herstellung von Arzneimitteln zur Verhinderung arterieller Restenosierung nach Angioplastie.

7. Verwendung von Verbindungen der Formel I gemäß Anspruch 1, 2 oder 3 zur Herstellung von Arzneimitteln zur Behandlung von Erkrankungen des venösen Systems.

8. Verwendung von Verbindungen der Formel I gemäß Anspruch 1, 2 oder 3 zur Herstellung von Arzneimitteln zur Verbesserung der Hämorheologie.

9. Verwendung von Verbindungen der Formel I gemäß Anspruch 1, 2 oder 3 zur Herstellung von Arzneimitteln zur Verhinderung oder Linderung Fibrinogen-assoziierter Krankheiten.

10. Verwendung von α,w-Dicarbonsäuren der vorliegenden Formel (I) für die Herstellung von Arzneimitteln mit Fibrinogen-senkender Wirkung, die bei der Zusatztherapie von Fibrinogen-assoziierten Dyslipidämien und Diabetes, sowie von Hypertension oder Gefäßverschlußkrankheiten eingesetzt werden.

11. Verwendung von Verbindungen der Formel I gemäß Anspruch 1, 2 oder 3 zur Herstellung von Arzneimitteln mit Fibrinogen-senkender Wirkung, die bei Zusatztherapie von Hypertension oder Gefäßverschlußkrankheiten eingesetzt werden.

## Claims

1. Use of α,ω-dicarboxylic acids corresponding to formula I 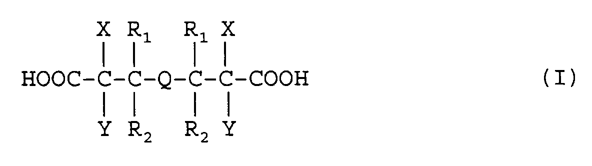 wherein
X and Y, which may be the same or different, denote hydrogen, halogen, C₁-C₆ alkyl, C₁-C₆ alkoxy, hydroxy, cyano, carboxyl, C₁-C₆ alkoxycarbonyl or carbamoyl,
R₁ and R₂, which may be the same or different, denote hydrogen or C₁-C₆ alkyl, and
Q denotes a linear saturated or unsaturated alkylene chain having 2 to 14 C atoms, wherein one or more C atoms may be replaced by cyclohexyl rings, phenyl or heterocyclic compounds,
and the *in vivo* hydrolysable carboxylic acid derivatives thereof, for the manufacture of medicaments having a fibrinogen-lowering action.

2. Use according to claim 1, characterised in that a compound corresponding to formula I is used,
wherein
X and Y, which may be the same or different, denote hydrogen or halogen,
R₁ and R₂, which may be the same or different, denote hydrogen or methyl, and
Q denotes a linear chain having 2 to 14 C atoms, a (CH₂)ₙ-cyclohexylidene-(CH₂)ₙ- group, a (CH₂)ₘ-phenylene-(CH₂)ₘ- group, a CH₂-CH=CH-phenylene-CH-CH-CH₂- group or a CH₂-CH=CH-CH₂-phenylene-CH₂-CH=CH-CH₂- group, wherein n represents the numbers 2, 3 or 4 and m the numbers 3 or 4, and the *in vivo* hydrolysable carboxylic acid derivatives thereof.

3. Use according to claim 1 or 2, characterised in that a compound corresponding to formula I is used, wherein X and Y each denote hydrogen, R₁ and R₂ each denote a methyl group and Q denotes a (CH₂)₁₀- group (compound A) and X and Y each denote chlorine, R₁ and R₂ each denote hydrogen and Q denotes a (CH₂)₈- group (compound B).

4. Use of compounds corresponding to formula I according to claim 1, 2 or 3 for the manufacture of medicaments for the lowering of fibrinogen in the blood and for the treatment and prevention of hyperfibrinogenaemia.

5. Use of compounds corresponding to formula I according to claim 1, 2 or 3 for the manufacture of medicaments for the prevention of fibrinogen-mediated alterations in the vessels.

6. Use of compounds corresponding to formula I according to claim 1, 2 or 3 for the manufacture of medicaments for the prevention of arterial restenosis after angioplasty.

7. Use of compounds corresponding to formula I according to claim 1, 2 or 3 for the manufacture of medicaments for the treatment of diseases of the venous system.

8. Use of compounds corresponding to formula I according to claim 1, 2 or 3 for the manufacture of medicaments for the improvement of the haemorheology.

9. Use of compounds corresponding to formula I according to claim 1, 2 or 3 for the manufacture of medicaments for the prevention or relief of fibrinogen-associated diseases.

10. Use of α,ω-dicarboxylic acids corresponding to the said formula I for the manufacture of medicaments having a fibrinogen-lowering action, which are used in the supplementary therapy of fibrinogen-associated dyslipidaemias and diabetes, and of hypertension or of diseases involving vascular occlusion.

11. Use of compounds corresponding to formula I according to claim 1, 2 or 3 for the manufacture of medicaments having a fibrinogen-lowering action, which are used in the supplementary therapy of hypertension or of diseases involving vascular occlusion.

## Revendications

1. Utilisation d'acides α,w-dicarboxyliques repondant à la formule I 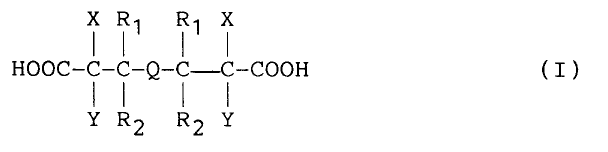 où
X et Y, qui peuvent être identiques ou différents, signifient l'hydrogène, un halogène, un groupe alkyle en C₁-C₆, alcoxy en C₁-C₆, hydroxy, cyano, carboxyle, alcoxycarbonyle en C₁-C₆ ou carbamoyle,
R₁ et R₂, qui peuvent être identiques ou différents, signifient l'hydrogène ou un groupe alkyle en C₁-C₆, et
Q signifie une chaîne alkylène linéaire, saturée ou insaturée, ayant de 2 à 14 atomes de carbone, dans laquelle un ou plusieurs atomes de carbone peuvent être remplacés par des noyaux cyclohexyle, par le phényle ou par des hérétocycles,
ainsi que de leurs dérivés acides carboxyliques hydrolysables *in vivo*, pour la préparation de médicaments ayant une action d'hypofibrinogénémie.

2. Utilisation selon la revendication 1, caractérisée en ce qu'on utilise un composé de formule I où
X et Y, qui peuvent être identiques ou différents, signifient l'hydrogène ou un halogène,
R₁ et R₂, qui peuvent être identiques ou différents, signifient l'hydrogène ou le groupe méthyle, et
Q signifie une chaîne linéaire ayant de 2 à 14 atomes de carbone, un groupe (CH₂)n-cyclohexylidène-(CH₂)ₙ, un groupe (CH₂)ₘ-phénylène-(CH₂)ₘ, un groupe CH₂-CH=CH-phénylène-CH=CH-CH₂ ou un groupe -CH₂-CH=CH-CH₂-phénylène-CH₂-CH=CH-CH₂, où n signifie le chiffre 2, 3 ou 4, et m signifie le chiffre 3 ou 4,
ainsi que de ses dérivés acides carboxyliques hydrolysables *in vivo*.

3. Utilisation selon la revendication 1 ou 2, caractérisée en ce qu'on utilise un composé de formule I où X et Y signifient chacun l'hydrogène, R₁ et R₂ signifient chacun un groupe méthyle, et Q signifie un groupe (CH₂)₁₀ (composé A), et X et Y signifient chacun le chlore, R₁ et R₂ signifient chacun l'hydrogène, et Q signifie un groupe (CH₂)₈ (composé B).

4. Utilisation de composés de formule I selon la revendication 1, 2 ou 3 pour la préparation de médicaments pour réduire le taux de fibrinogène dans le sang, ainsi que pour le traitement et la prévention de l'hyperfibrinogénémie.

5. Utilisation de composés de formule I selon la revendication 1, 2 ou 3 pour la préparation de médicaments pour empêcher des modifications des vaisseaux induites par le fibrinogène.

6. Utilisation de composés de formule I selon la revendication 1, 2 ou 3 pour la préparation de médicaments pour empêcher une resténose artérielle après une angioplastie.

7. Utilisation de composés de formule I selon la revendication 1, 2 ou 3 pour la préparation de médicaments pour le traitement des maladies du système veineux.

8. Utilisation de composés de formule I selon la revendication 1, 2 ou 3 pour la préparation de médicaments pour l'amélioration de l'hémorhéologie.

9. Utilisation de composés de formule I selon la revendication 1, 2 ou 3 pour la préparation de médicaments pour empêcher ou atténuer les maladies associées au fibrinogène.

10. Utilisation d'acides α,w-dicarboxyliques répondant à la présente formule I pour la préparation de médicaments ayant une action d'hypofibrinogénémie, qu'on utilise lors de la thérapie d'appoint de la dyslipidémie associée au fibrinogène et du diabète, ainsi que de l'hypertension ou des maladies d'occlusion vasculaire.

11. Utilisation de composés de formule I selon la revendication 1, 2 ou 3 pour la préparation de médicaments ayant une action d'hypofibrinogénémie, qu'on utilise pour la thérapie de l'hypertension ou de maladies d'occlusion vasculaire.
